# EUROPEAN PATENT APPLICATION

(11) **EP 1 213 007 A2**
(43) Date of publication of application: **12.06.2002**
(21) Application number: 01310187.8
(22) Date of filing: 05.12.2001
(51) Int. Cl.: A61K 7/00, A61P 17/10

(54) **Personal care formulations**

(30) Priority: 06.12.2000 US 731342
(71) Applicant: Johnson & Johnson Consumer Companies, Inc., Skillman, New Jersey 08558 (US)
(72) Inventor: Wiegand, Benjamin, Newton, PA 18940 (US); McCulloch, Laura, Kings Somborne, Hampshire, SO20 6PE (GB); Lukenbach, Elvin, Flemington, NJ 08822 (US)
(74) Representative: Mercer, Christopher Paul

(57) **Abstract**

The invention relates to a method of depositing a benefit agent on a keratinous surface, said method comprising topically applying to said surface an effective amount of a ringing gel composition comprising (a) a surfactant phase; (b) an oil phase; and (c) a benefit agent.

## Description

### Cross Reference to Related Application

This application is related to co-pending European Patent Application No. 00945731.8, (WO00/76460), the disclosure of which is hereby incorporated by reference.

### Field of the Invention

The present invention relates to methods for depositing benefit agents to keratinous surfaces, such as, the skin, hair and nails of humans or animals.

### Background of the Invention

Dispersing oil, oil-soluble materials and particulates in aqueous shampoo and body wash formulations has presented problems. To prevent the oil phase separating, it must either be: (A) emulsified which involves dispersing the oil as colloidal single droplets; (B) microemulsified which involves forming a micellar solution with oil incorporated into surfactant micelles; (C) suspended in a structured surfactant system which typically comprises a dispersion of a surfactant mesophase in aqueous electrolyte; or (D) incorporated into a water soluble solid, pasty or gelatinous composition.

With the exception of microemulsions which are clear, thermodynamically stable, micellar solutions, the foregoing systems are necessarily opaque and contain the oil dispersed in a relatively coarse form which does not deposit satisfactorily on skin or hair. However, microemulsions are difficult to formulate using the surfactants which are most effective in body wash and other personal care formulations and contain relatively low concentrations of surfactant.

Hexagonal gels (M phase) have been referred to in the prior art as cleaning compositions, for example, Great Britain Patent No. 2,170,055 and European Patent No. 1,153,837. Colloidal gels formed with gelling agents such as synthetic polymers or gelatin have also been suggested, for example, by U.S. Patent No. 4,465,663. However, these compositions cannot be readily dissolved in water to form microemulsions. They are moreover usually opaque and of an unattractive appearance and often require the presence of solvents such as glycols which add to the cost and are environmentally undesirable.

The use of a type of ringing gel to suspend oil for cosmetic or pharmaceutical applications is described in U.S. Patent No. 4,026,818, but the formulation requires the presence of hydroxylic solvents and utilizes a surfactant system which is unsuitable for shampoo applications. European Patent No. 598,335 describes the use of various cubic phases, including I₁ phases as laundry prespotters and for other cleaning formulations. It does not suggest how such phases can be used to suspend oil or form microemulsions. Normally, attempts to suspend oil in surfactant mesophases result in coarse droplets of oil being suspended in the aqueous phase of a structured surfactant. As described in WO00/76460, it has been discovered that oil may be stably incorporated into the structure of an µ phase to form a clear, gel-like composition which contains higher concentrations of surfactant and oil than conventional microemulsions, but which dissolves in water to form a microemulsion. The novel all-in-1 compositions also form microemulsions on heating.

It has also been discovered that such compositions may be used to deposit benefit agents onto keratinous substrates, such as the skin, hair and nails of a human or animal, even after rinsing the composition off of the skin. This finding of a "2 in 1" composition, where the product cleanses as well as leaves a particular benefit agent behind is novel, as one would expect that the cleansing surfactants present in the composition would remove all of the benefit agent from the surface. In particular, this result is unexpected as compositions of this type have been described in Great Britain Patent Application No. 99133082, filed June 10, 1999, as being useful in the removal of oils and particulate material in laundry applications.

### Summary of Invention

The invention relates to a method of depositing a benefit agent on a keratinous surface, said method comprising topically applying to said surface an effective amount of a ringing gel composition comprising (a) a surfactant phase; (b) an oil phase; and (c) a benefit agent.

It has been discovered that the compositions according to the invention are especially effective at depositing anti-acne agents to the skin of a mammal. Accordingly, in another embodiment, the invention relates to a method for treating acne of a mammal comprising topically applying, to the affected area of the skin, an effective amount of ringing gel composition comprising (a) a surfactant phase; (b) an oil phase; and (c) an anti-acne agent.

It has also been discovered that the compositions of the invention are particularly useful as "2 in 1" composition, where the composition cleanses as well as leaves a particular benefit agent behind, even after rinsing the composition off of the skin. Accordingly, in another embodiment, the invention relates to a method of cleansing and delivering a benefit agent to hair, skin or nails of a mammal, comprising topically applying to a desired location an effective amount of a ringing gel composition comprising (a) a surfactant phase; (b) an oil phase; and (c) a benefit agent.

### Detailed Description

As used herein, "alkyl" means an unsubstituted carbon-containing chain which may be straight, branched or cyclic, preferably straight or branched, more preferably straight; saturated, monounsaturated (i.e., one double or triple bond in the chain), or polyunsaturated (i.e., two or more double bonds in the chain; two or more triple bonds in the chain; one or more double and one or more triple bonds in the chain), preferably saturated.

As used herein, "topical application" means directly laying on or spreading on outer skin, hair or nails.

As used herein, "effective amount" means an amount that is high enough to deliver a desired skin, hair or nail benefit or to modify a certain condition to be treated, but is low enough to avoid serious side effects, at a reasonable risk to benefit ratio within the scope of sound medical judgment.

As used herein, the "hydrophilic: lipophilic balance" or "HLB"value is used as a measure of the hydrophile-lipophile balance of an ingredient or combination of ingredients. In the context of this invention, surfactants are assigned a number which indicates the relative affinities of the surfactants for water and oil, respectively and correlates with their effectiveness as emulsifiers. HLB value can easily be calculated for alcohol ethoxylates since it is one-fifth of the weight percent of ethylene oxide, based on the total mole weight. Other surfactants can be assigned equivalent values by applying more complicated formula or by measuring their relative affinity for water and oil. An HLB value of 20 represents completely water-soluble oil, insoluble surfactant, while an HLB value of 0 represents a completely oil soluble and water insoluble surfactant. Just as surfactants and emulsifiers have a HLB value, ingredients, such as the oils used in the ringing gel compositions according to the invention, have an HLB value with more polar oils, having a higher HLB than less polar oils.

As used herein, the term "benefit agent" includes any active ingredient that is to be delivered into and/or onto a keratinous surface, such as, the skin, hair or nail at a desired location.

All references herein to the formation or existence of specific phases or structures and to viscosity are to be construed, unless the context requires otherwise, as references to their formation or existence at 20°C.

As used herein, all percentages are by weight unless otherwise specified.

The invention relates to a method of depositing a benefit agent on a keratinous surface, said method comprising topically applying to said surface an effective amount of a ringing gel composition comprising (a) a surfactant phase; (b) an oil phase; and (c) a benefit agent. The keratinous surface is selected from the skin, hair, and/or nails of a human or animal.

The surfactants are preferably selected to provide a ringing gel, over a comparatively broad surfactant concentration range, e.g., more than a 5 percent or greater which range typically lies above 10 percent by weight total based on the weight of the composition, e.g., between 10 percent and 50 percent by weight surfactant usually between 20 percent and 45 percent. In a preferred embodiment, the surfactants are selected to provide a ringing gel which melts above 30°C, e.g., above 35°C, most preferably above 40°C. Preferably the ringing gel melts at a temperature substantially below 100°C, e.g., below 90°C, more preferably below 80°C, most preferably below 70°C, especially below 60°C, typically below 55°C, usually below 50°C.

In a preferred embodiment, the surfactant phase has a mean HLB based on the molar proportions of the components between about 10 to about 15, more preferably from about 11 to about 14. Preferred surfactants useful in the surfactant phase are selected from anionic, amphoteric and nonionic surfactants.

Suitable nonionic surfactants include the following:

Alcohol ethoxylates, alkyl phenol ethoxylates, fatty acid ethoxylates, fatty acid monoalkylolamide ethoxylates, fatty alcohol propoxylates, fatty amine alkoxylates and fatty acid glyceryl ester ethoxylates. Other non-ionic compounds suitable for inclusion in compositions of the present invention include mixed ethylene oxide propylene oxide alkoxylates, low relative molecular mass polyethylene glycols, e.g., PEG600 and PEG200, ethylene glycol monoesters, amine oxides and alkyl polyglycosides, alkyl sugar esters including alkyl sucrose esters and alkyl oligosaccharide ester, alkyl capped polyvinyl alcohol and alkyl capped polyvinyl pyrrolidone. Generally, the chain lengths of the alcohols, fatty acids, fatty amines, and alkyl groups mentioned above range from 8 to 22 and the degree of ethoxylation and/or propoxylation, where appropriate, ranges from 2 to 20 moles of ethylene oxide and/or propylene oxide.

In a particularly preferred embodiment, the nonionic surfactant is selected from C₁₂-C₁₄ alcohol ethoxylates have from about 2 to about 12 ethylene oxide groups. Also preferred are C₈-C₁₀ alkyl polyglucosides.

Suitable anionic surfactants for use in the ringing gel compositions according to the invention may be selected from the following: alkyl sulfates; alkyl ether sulfates; alkyl monoglyceryl ether sulfates; alkyl monoglyceride sulfates; alkyl monoglyceride sulfonates; alkyl sulfonates; alkylaryl sulfonates; alkyl sulfosuccinates; alkyl ether sulfosuccinates; alkyl sulfosuccinamates; alkyl amidosulfosuccinates; alkyl carboxylates; alkyl ether carboxylates; alkyl amidoethercarboxylates; alkyl succinates; fatty acyl sarcosinates; fatty acyl amino acids; fatty acyl taurates; fatty alkyl sulfoacetates; alkyl phosphates; alkyl isethionates, and mixtures thereof. Generally, the alkyl and acyl groups mentioned above have chain lengths ranging from 8 to 22 carbon atoms and the degree of ethoxylation, where appropriate, ranges from 2 to 20 moles of ethylene oxide. Many different salts of the anionic surfactants can be used, including, bot not limited to the alkali metals (potassium, lithium, sodium, etc.) as well as magnesium, calcium, ammonium, triethanolamine, and other amino moieties. Particularly preferred anionic surfactants include C₈-C₂₂ alkyl ether carboxylic acids, C₈-C₂₂ alkyl sulfates and C₈-C₂₂ alkyl ethoxy sulfates. Examples of preferred anionic surfactants include, but are not limited to, C₈ alkyl ether carboxylic acid, C₈-C₁₀ alkyl sulphate, C₁₂-C₁₄ alkyl ether sulphate having from about 2 to about 5 ethylene oxide groups.

The composition may contain amphoteric surfactants including, but not limited to alkyl amphocarboxylates, alkyl betaines, amidoalkyl betaines, amidoalkyl sultaines, alkyl amphophosphates, alkyl phosphobetaines, alkyl pyrophosphobetaines, alkyl sulfobetaines, carboxyalkyl alkyl polyamines. Generally, the alkyl groups mentioned above have chain lengths ranging from 3 to 22 carbon atoms.

The proportion of oil in the ringing gel compositions according to the invention, preferably ranges from about 5 percent to about 50 percent, more preferably from about 10 percent to about 35 percent and most preferably from about 15 percent to about 25 percent by weight, based on the weight of the composition.

The oil phase is preferably selected from the group consisting of mineral oil (e.g., a low molecular weight petroleum ether), alkyl esters, silicone oils, and mixtures thereof. Examples of suitable mineral oils include maleated soybean oil, light mineral oil, e.g., having a viscosity of less than about 20 cps, heavy mineral oil, e.g., having a viscosity above 20 cps, triglycerides, such as, sunflower triglycerides, and capric/caprylic triglycerides.

Examples of suitable silicone oils include organo-substituted polysiloxanes, which are either linear or cyclic polymers of monomeric silicone/oxygen monomers and which nonexclusively include cetyl dimethicone; cetyl triethylammonium dimethicone copolyol phthalate; cyclomethicone; dimethicone copolyol; dimethicone copolyol lactate; hydrolyzed soy protein/dimethicone copolyol acetate; silicone quaternium 13; stearalkonium dimethicone copolyol phthalate; stearamidopropyl dimethicone; and mixtures thereof. Additional silicone oils suitable for use in the compositions according to the invention are those known in the art for use in hair and skin care compositions and are taught, for example, by U.S. Reissue Patent No. 34,584.

Examples of suitable alkyl esters for use in the ringing gel composition of this invention include those liquid esters that either possess a structural means for ensuring its liquidity or are heterogeneous in nature. Examples of such structural means include the presence of "interruptions", such as: 1) chain branching; 2) olefinic unsaturation; 3) the presence of either a polyether or a monoalkoxylate in the structure; or 4) the presence of a substituent, e.g. an ethoxylated or propoxylated moiety, bonded between the acid group and the alcohol group. By "heterogeneity," it is meant that the lipophilic component is comprised of a mixture of compounds that vary in the number of carbon atoms in their respective chains.

Examples of suitable esters nonexclusively include:
a) a branched C₅ to C₂₂ alkyl alcohol ester of an aromatic acid;
b) a straight-chained or branched C₅ to C₂₂ alkyl acid esters of optionally ethyoxylated/propoxylated polyols having from about 3 carbon atoms to about 7 carbon atoms;
c) branched C₅ to C₂₂ alkyl alcohol esters of branched polyacids;
d) branched or straight-chained C₅ to C₂₂ alkyl acid esters of branched and/or unsaturated C₅ to C₂₂ alkyl alcohols;
e) branched or unsaturated C₅ to C₂₂ alkyl alcohol esters of an acid selected from the group consisting of adipic acid, succinic acid, maleic acid, sebacic acid, and mixtures thereof
f) polyether interrupted fatty acid esters;
g) benzoic acid ester of heterogeneous alcohols having from about 8 carbon atoms to about 22 carbon atoms; and
h) mixtures thereof,
with straight-chained or branched C₅ to C₂₂ alkyl acid esters of optionally ethyoxylated/propoxylated polyols, benzoic acid esters of heterogeneous alcohols, and mixtures thereof being particularly preferred.

Suitable alkyl esters are described, for example, in copending application nos. 09/604,563 and 09/604,449, filed concurrently on June 27, 2000, the disclosures of which are hereby incorporated by reference.

For example, suitable alkyl esters include straight-chained or branched C₅ to C₂₂ alkyl acid esters of optionally ethyoxylated/propoxylated polyols, wherein the polyols contain from about 3 carbon atoms to about 7 carbon atoms. Preferably, if the polyol creates a branching point, then the acid group may be straight-chained. Suitable acids used to form these esters typically have from about 8 carbon atoms to about 22 carbon atoms, and preferably from about 8 carbon atoms to about 18 carbon atoms, and most preferably from about 8 carbon atoms to about 12 carbon atoms, and are either saturated or unsaturated, with octanoic acid, capric acid, and mixtures thereof being preferred. Such suitable acids are either straight-chained or branched, and are preferably aliphatic. Suitable polyols used to form these esters typically have from about 3 carbon atoms to about 30 carbon atoms, and preferably from about 3 carbon atoms to about 7 carbon atoms. Examples of such suitable polyols nonexclusively include neopentyl alcohol; polyglycerol, e.g. diglycerol, triglycerol, hexaglycerol, and decaglycerol, wherein the polyglycerol may contain from about 2 to about 10 glycerol groups; glycerin; sorbitan; methyl glucose; trimethylolpropane; and mixtures thereof. Neopentyl alcohol, glycerin, trimethylolpropane, and mixtures thereof are the preferred polyols. Examples of suitable esters nonexclusively include pentaerythritol tetraoctanoate; trimethylolpropane trioctanoate; trioctanoin; pentaerythrityl tetrapelargonate; sorbitan trioleate; caprylic/capric triglyceride; neopentyl alcohol tetraoctanoate, and mixtures thereof, with caprylic/capric triglyceride; pentaerythritol tetraoctanoate; trimethylolpropane trioctanoate; and pentaerythrityl tetrapelargonate being more preferred.

Another suitable ester includes the branched C₅ to C₂₂ alkyl alcohol esters of branched polyacids such as the tri-esters, tetra-esters, penta-esters, and mixtures thereof. An example of such a polyacid is citric acid. Suitable alkyl alcohols for creating these esters are optionally substituted, e.g., ethoxylated or propoxylated, contain from about 3 carbon atoms to about 22 carbon atoms, and preferably from about 3 carbon atoms to about 8 carbon atoms, and are either straight-chained or branched, with the branching being preferred. These alcohols may either be primary or secondary, and may either be saturated or unsaturated, with saturated being preferred for stability reasons. Specific examples of suitable esters nonexclusively include trioctyldodecyl citrate; triisopropylcitrate; and mixtures thereof.

Another suitable ester includes the branched or straight-chained C₅ to C₂₂ alkyl acid esters of branched or unsaturated alkyl alcohols wherein the alkyl group of the alcohol has from about 1 carbon atoms to about 18 carbon atoms, and preferably from about 4 carbon atoms to about 10 carbon atoms, provided that the total number of carbon atoms in the ester is at least about 8. Suitable acids for use in making these esters typically have from about 2 carbon atoms to about 22 carbon atoms, and preferably from about 5 carbon atoms to about 10 carbon atoms. However, if the number of acid carbon atoms exceeds the number of carbon atoms in the alcohol, then the acid preferably contains from about 8 carbon atoms to about 18 carbon atoms and the alcohol preferably contains from about 1 carbon atom to about 8 carbon atoms. If the number of acid carbon atoms is less than the number of carbon atoms in the alcohol, then the acid preferably contains from about 2 carbon atoms to about 8 carbon atoms and the alcohol preferably contains from about 8 carbon atoms to about 18 carbon atoms. Preferably, either: 1) the alcohol group or the acid group has branching and/or unsaturation, i.e. both the alcohol and the acid are not straight-chained; or 2) the ester possesses an asymmetrical alkyl distribution. By "asymmetrical alkyl distribution," it is meant that the ester is made from, for example, a short chain alcohol, i.e. having from about 1 carbon atom to about 8 carbon atoms, and a long chain acid, i.e., having greater than about 8 carbon atoms, such as, e.g. butyl stearate, or less preferably the ester is made from, a long chain alcohol, i.e. having greater than about 8 carbon atoms, and a short chain acid, i.e. having from about 1 carbon atom to about 8 carbon atoms. Examples of such suitable esters nonexclusively include tridecyl neopentanoate, isostearyl palmitate, cetyl ricinoleate, cetyl octanoate, isononyl isononanoate, butyl stearate, octyldodecyl soyate, tridecyl erucate, octyldodecyl erucate/eicosil erucate, and mixtures thereof, with cetyl octanoate, isostearyl palmitate, isononyl isononanoate, and mixtures thereof and being preferred.

Another suitable ester includes the branched or unsaturated C₅ to C₂₂ alkyl alcohol esters of an acid selected from the group consisting of adipic acid, succinic acid, maleic acid, sebacic acid, and mixtures thereof. The alcohol of these esters, which has from about 3 carbon atoms to about 18 carbon atoms, and preferably from about 3 carbon atoms to about 8 carbon atoms, is preferably branched or unsaturated. Examples of such suitable alcohol esters nonexclusively include isopropyl myristate, diisopropyl adipate, dioctyl sebacate, dioctyl succinate, dioctyl maleate, diisostearyl adipate, diethyl sebacate, and mixtures thereof.

Preferred oils for use in the compositions according to the invention include maleated soybean oil, light mineral oil, e.g., having a viscosity of less than about 20cps, heavy mineral oil, e.g., having a viscosity above 20cps, isopropyl myristate, triglycerides, such as, sunflower triglycerides, and capric/caprylic triglycerides.

In a preferred embodiment, the ringing gel composition according to the invention comprises (a) from about 60 to about 95% by wt. of the surfactant phase, based on the total composition; and (b) from about 5 to about 40% by wt. of oil the phase, based on the total composition.

The amounts and types of surfactant and oil set forth above provide a general description of suitable surfactants and oils for use in the ringing gel composition of the invention. It should be understood, however, that the preferred surfactant system and the preferred oil may vary depending upon, for example, the ability of the benefit agent to penetrate through the skin, hair or nail, the specific benefit agent chosen, the particular benefit desired, the sensitivity of the user to the benefit agent, the health condition, age, and skin, hair, and/or nail condition of the user, and the like. Generally, however, it has been discovered that a lower level of amphoteric surfactant in the surfactant phase leads to improved deposition of the benefit agent. In one embodiment of the invention, it is preferred that the ratio of amphoteric surfactant to total surfactant range from about 0.05 to about 0.6 and the ratio of anionic surfactant to total surfactant range from about 0.1 to about 0.9. Further, the nature of the oil used in the oil phase will also significantly affects deposition of the benefit agent. For example, the higher the viscosity and/or the lower the HLB of the oil phase, the more improved the deposition. Accordingly, in another embodiment of the invention, it is preferred that the viscosity of the oil phase range from 1 to 500 centistokes, more preferably from about 10 centistokes to about 100 centistokes and the HLB ranges from about 3 to about 18, more preferably from about 8 to 11.

It has also been discovered that the amount of water present in the ringing gel composition can affect deposition of the benefit agent. For example, in one embodiment of the invention the amount of water present in the ringing gel composition according to the invention, preferably ranges from about 20 to about 70 wt.%, more preferably, from about 30 to about 50 wt.%, based on the total composition.

The amount of benefit agent to be combined with the cleansing composition or the emulsion may vary depending upon, for example, the ability of the benefit agent to penetrate through the skin, hair or nail, the specific benefit agent chosen, the particular benefit desired, the sensitivity of the user to the benefit agent, the health condition, age, and skin, hair, and/or nail condition of the user, and the like. In sum, the benefit agent is used in a "safe and effective amount," which is an amount that is high enough to deliver a desired skin, hair or nail benefit or to modify a certain condition to be treated, but is low enough to avoid serious side effects, at a reasonable risk to benefit ratio within the scope of sound medical judgment. Typically the benefit agent is present in the composition in an amount, based upon the total weight of the system, from about 0.01 percent to about 10.0 percent, and preferably from about 0.5 percent to about 5 percent, and more preferably from about 1.0 percent to about 2.5 percent.

The benefit agents useful herein may be categorized by their therapeutic benefit or their postulated mode of action. However, it is to be understood that the benefit agents useful herein may, in some circumstances, provide more than one thereapeutic benefit or operate via greater than one mode of action. Therefore, the particular classifications provided herein are made for the sake of convenience and are not intended to limit the benefit agents to the particular application(s) listed. In addition, the compounds, which are identified below as being suitable for use as benefit agents, may be used in an amount over and above the amount that they may be used for other purposes in the cleansing composition/cleansing system. Examples of suitable benefit agents for use in the present invention, include but are not limited to, those described in copending U.S. Patent Application Nos. 09/604,564 and 09/604,449, filed concurrently on June 27, 2000, the disclosure of which is hereby incorporated by reference.

Oil soluble benefit agents may be dissolved in the oil, including antiseptics, styptics, anti-dandruff agents such as zinc omadine (zinc pyrithione) and selenium disulphide, proteins, emollients such as lanolin, isopropyl myristate, glyceryl isostearate, or propylene glycol distearate, anti-irritants, anti-inflammatories, anti-microbial agents, sensates, anti-puritics, skin protectants, keratolytics, hair growth actives, anti-aging actives, exfoliants, anti-cellulite, anti- pigmentation actives, organic sunscreens, antioxidants, natural extracts, dyes, perfumes, fragrances, and waxes.

In some cases, the oil present in the oil phase of the ringing gel composition can act simultaneously as a benefit agent. For example, emollients, such as, isopropyl myristate, is preferred oil for the oil phase but also has the additive effect of providing skin emolliency, softening and/or moisturizing effects. Other examples of oils which can act as a benefit agent are the silicone oils discussed above.

Examples of suitable benefit agents include, but are not limited to, depigmentation agents; reflectants; detangling/wet combing agents; film forming polymers; humectants; amino acids and their derivatives; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents, antihistamines such as Mandragora Vernalis, Tanacetum Parthenium and the like; antiinfectives such as Acacia Catechu, Aloe Barbadensis, Convallaria Majalis, Echinacea, Eucalyptus, Mentha Piperita, Rosa Canina, Sassafras Albidum, and the like; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; skin firming agents, hair conditioners; hair softeners; hair moisturizers; vitamins; tanning agents; skin lightening agents; antifungals such as Centaurea Cyanus, Kalmia Latifolia and antifungals for foot preparations; depilating agents; shaving preparations; external analgesics; perfumes; fragrances; counterirritants; hemorrhoidals; insecticides; poison ivy products; poison oak products; burn products; anti- diaper rash agents; prickly heat agents; make-up preparations; vitamins; amino acids and their derivatives; herbal extracts; retinoids; flavenoids; sensates; anti-oxidants; skin conditioners; hair lighteners; chelating agents; cell turnover enhancers; coloring agents; pigments; sunscreens, those active ingredients disclosed in United States Patent No. 6,063,397, which is incorporated herein by reference, anti-edema agents, collagen enhancers, and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, lycopene including "Lyc-o-Pen" available from LycoRed Natural Products Industries, Ltd., oat oil, chamomile, and mixtures thereof.

Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like. These antipruritics may be used in an amount, based upon the total weight of the cleansing composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation and colloidal oatmeals available from Quaker.

Examples of suitable reflectants nonexclusively include mica, alumina, calcium silicate, glycol dioleate, glycol distearate, silica, sodium magnesium fluorosilicate, and mixtures thereof.

Suitable detangling/wet combing agents nonexclusively include polyquaternium-10, hydroxypropyltrimonium guar, dioleoylamidoethyl hydroxyethylmonium methosulfate, di-(soyoylethyl) hydroxyethylmonium methosulfate, hydroxyethyl behenamidopropyl dimonium chloride, olealkonium chloride, polyquaternium-47, stearalkonium chloride, tricetylmonium chloride, and mixtures thereof.

Suitable film forming polymers include those that, upon drying, produce a substantially continuous coating or film on the hair, skin, or nails. Nonexclusive examples of suitable film forming polymers include acrylamidopropyl trimonium chloride/acrylamide copolymer; corn starch/ acrylamide/ sodium acrylate copolymer; polyquatemium-10; polyquaternium-47; polyvinylmethylether/maleic anhydride copolymer; styrene/acrylates copolymers; and mixtures thereof.

Commercially available humectants which are capable of providing moisturization and conditioning properties to the cleansing composition are suitable for use in the present invention. The humectant is preferably present in an amount of from about 0 percent to about 10 percent, more preferably from about 0.5 percent to about 5 percent, and most preferably from about 0.5 percent to about 3 percent, based on the overall weight of the composition. Examples of suitable humectants nonexclusively include: 1) water soluble liquid polyols selected from the group comprising glycerine, propylene glycol, hexylene glycol, butylene glycol, pentylene glycol, dipropylene glycol, and mixtures thereof; 2) polyalkylene glycol of the formula I.:

HO-(R"O)_{b}-H I.

wherein R" is an alkylene group having from about 2 to about 4 carbon atoms and b is an integer of from about 1 to about 10, such as PEG 4; 3) polyethylene glycol ether of methyl glucose of formula II.:

CH₃-C₆H₁₀O₅-(OCH₂CH₂)_{c}-OH II.

wherein c is an integer from about 5 to about 25;
4) urea; 5) fructose; 6) glucose; 7) honey; 8) lactic acid; 9) maltose; 10) sodium glucuronate; and 11) mixtures thereof, with glycerine being the preferred humectant.

Suitable amino acid agents include amino acids derived from the hydrolysis of various proteins as well as the salts, esters, and acyl derivatives thereof. Examples of such amino acid agents nonexclusively include amphoteric amino acids such as alkylamido alkylamines, i.e. stearyl acetyl glutamate, capryloyl silk amino acid, capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; amino acids such as aspartic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline; lysine; silk amino acids, wheat amino acids; and mixtures thereof

Suitable proteins include those polymers that have a long chain, i.e. at least about 10 carbon atoms, and a high molecular weight, i.e. at least about 1000, and are formed by self-condensation of amino acids. Nonexclusive examples of such proteins include collagen, deoxyribonuclease, iodized corn protein; milk protein; protease; serum protein; silk; sweet almond protein; wheat germ protein; wheat protein; alpha and beta helix of keratin proteins; hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Examples of suitable vitamins nonexclusively include vitamin B complex; including thiamine, nicotinic acid, biotin, pantothenic acid, choline, riboflavin, vitamin B6, vitamin B12, pyridoxine, inositol, carnitine; vitamins A,C,D,E,K and their derivatives such as vitamin A palmitate and pro-vitamins, e.g. (i.e. panthenol (pro vitamin B5) and panthenol triacetate) and mixtures thereof.

Examples of suitable antibacterial agents nonexclusively include bacitracin, erythromycin, neomycin, tetracycline, chlortetracycline, benzethonium chloride, phenol, and mixtures thereof.

Examples of suitable skin emollients and skin moisturizers nonexclusively include mineral oil, lanolin, vegetable oils, isostearyl isostearate, glyceryl laurate, methyl gluceth-10, methyl gluceth-20 chitosan, and mixtures thereof.

Examples of suitable hair conditioners nonexclusively include quaternized compounds such as behenamidopropyl PG-dimonium chloride, tricetylmonium chloride, dihydrogenated tallowamidoethyl hydroxyethylmonium methosulfate, and mixtures thereof as well as lipophilic compounds like cetyl alcohol, stearyl alcohol, hydrogenated polydecene, and mixtures thereof.

An example of a suitable hair softener nonexclusively includes silicone compounds, such as those that are either non-volatile or volatile and those that are water soluble or water insoluble. Examples of suitable silicones include organo-substituted polysiloxanes, which are either linear or cyclic polymers of monomeric silicone/oxygen monomers and which nonexclusively include cetyl dimethicone; cetyl triethylammonium dimethicone copolyol phthalate; cyclomethicone; dimethicone copolyol; dimethicone copolyol lactate; hydrolyzed soy protein/dimethicone copolyol acetate; silicone quaternium 13; stearalkonium dimethicone copolyol phthalate; stearamidopropyl dimethicone; and mixtures thereof. Suitable silicone oils are those known in the art for use in hair and skin care compositions and are taught, for example, by U.S. Reissue Patent No. 34,584.

Examples of suitable hair moisturizers nonexclusively include panthenyl ethyl ether, phytantriol, and mixtures thereof.

Examples of sunscreen agents nonexclusively include benzophenones, bornelone, butyl paba, cinnamidopropyl trimethyl ammonium chloride, disodium distyrylbiphenyl disulfonate, paba, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate O, red petrolatum, and mixtures thereof.

An example of a suitable tanning agent nonexclusively includes dihydroxyacetone.

Examples of skin lightening agents nonexclusively include hydroquinone, catechol and its derivatives, ascorbic acid and its derivatives, and mixtures thereof.

Examples of suitable insecticides (including insect repellents, anti-scabies and anti-lice treatments) nonexclusively include permethrin, pyrethrin , piperonyl butoxide, imidacloprid, N,N-diethyl toluamide, which refers to the material containing predominantly the *meta* isomer, i.e., N,N-diethyl-*m*-toluamide, which is also known as DEET; compounds of the formula below: 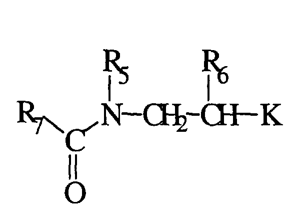 wherein
R₅ is a branched or unbranched alkyl group having about 1 to about 6 carbon atoms;
R₆ is H, methyl or ethyl;
R₇ is a branched or unbranched alkyl or alkoxy group having from about 1 to about 8 carbon atoms; and
K is a -CN or a -COOR₈ group, wherein
R₈ is a branched or unbranched alkyl group having from about 1 to about 6 carbon atoms,
natural or synthetic pyrethroids, whereby the natural pyrethroids are contained in pyrethrum, the extract of the ground flowers of *Chrysanthemum cinerariaefolium* or *C coccineum;* and mixtures thereof. Within the structure of Formula III. are ethyl 3-(N-butylacetamido)propionate, wherein R₇ is a CH₃ group, R₅ is an n-butyl group, R₆ is H, K is COOR₈ and R₈ is ethyl, which is available commercially from Merck KGaA of Darmstadt, Germany under the name, "Insect Repellent 3535."

An example of an anti fungal for foot preparations nonexclusively includes tolnaftate.

Examples of suitable depilating agents nonexclusively include calcium thioglycolate, magnesium thioglycolate, potassium thioglycolate, strontium thioglycolate, and mixtures thereof.

Examples of suitable external analgesics and local anesthetics nonexclusively include benzocaine, dibucaine, benzyl alcohol, camphor, capsaicin, capsicum, capsicum oleoresin, juniper tar, menthol, methyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone, Fragaria Vesca, Matricaria Chamomilla, and Salvia Officinalis.

Examples of suitable hemorrhoidal products nonexclusively include the anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Most preferred benefit agents nonexclusively include anti-acne agents, such as salicylic acid, DMAE, soy and derivatives thereof, colloidal oatmeal, sulfonated shale oil, olive leaf, elubiol, 6-(1-piperidinyl)-2,4-pyrimidinediamine-3-oxide, finasteride, ketoconazole, salicylic acid, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One preferred type of benefit agent includes those therapeutic components that are effective in the treatment of dandruff, seborrheic dermatitis, and psoriasis as well as the symptoms associated therewith. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

Another embodiment of the present invention is directed to a method for depositing a benefit agent onto the skin, hair and/or nails comprised of applying either the above-described ringing gel composition with an effective amount of a benefit agent to a desired location on a human or animal. While the frequency and amount of the benefit agent-containing cleaning system to be applied will depend upon, for example, the type and amount of benefit agent available, the intended usage of the final composition, i.e. therapeutic versus maintenance regimen, the amount and type of detergent present, and the sensitivity of the individual user to the composition/emulsion, typically the benefit agent-containing cleaning system of the present invention should be topically applied to affected body parts at regular intervals, and preferably from about 2 to about 14 times per week. More preferably, the composition/emulsion is applied more frequently during the initial stages of treatment, e.g. from about 5 to about 7 times per week until the desired effect is achieved, then less frequently when maintenance is desired, e.g. from about 2 to about 5 times per week.

Another preferred embodiment of the present invention is directed to a method for treating acne, comprising topically applying the above-described ringing gel composition comprising an effective amount of an anti-acne agent to the skin of an animal or human at a desired area.

Examples of suitable anti-acne agents include, but are not limited to topical retinoids (tretinoin, isotretinoin, motretinide, adapalene, tazarotene, azelaic acid, retinol); salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, arnica, artemisia capillaris, asiasarum root, birrh, calendula, chamomile, cnidium, comfrey, fennel, galla rhois, hawthorn, houttuynia, hypericum, jujube, kiwi, licorice, magnolia, olive, peppermint, philodendron, salvia, sasa albo-marginata; imidazoles such as ketoconazole and elubiol, and those described in Gollnick, H et al. 196(1) Dermatology Sebaceous Glands, Acne and Related Disorders, 119-157 (1998), which is incorporated by reference herein, and mixtures thereof.

Preferred anti-acne agents include salicylic acid, benzoyl peroxide, retinol, elubiol, antibiotics, and salicylic acid, with retinol and tretinoin being most preferred.

Suitable amount of anti-acne agents include, based upon the total weight of the described cleaning system, from about 0.01 percent to about 10 percent, and preferably from about 0.5 percent to about 2.5 percent.

As discussed above, it has been discovered that the compositions of the invention are particularly useful as "2 in 1" composition, where the composition cleanses as well as leaves a particular benefit agent behind. Accordingly, in another embodiment, the ringing gel compositions discussed above can be used in a method for cleansing and delivering a benefit agent to hair, skin or nails of a mammal.

Preferably, the ringing gel compositions according to the invention comprise from about 10 percent to 80, more preferably from about 20 percent to about 70 percent by weight, based on the total composition, and most preferably from about 20 to about 50 wt. % water, based on the total composition.

The compositions of the present invention may also include one or more optional ingredients including a pearlescent or opacifying agent, a thickening agent, secondary conditioners, humectants, builders, chelating agents, and additives which enhance their appearance, feel and fragrance, such as colorants, pigments, fragrances, preservatives, pH adjusting agents, skin conditioners, sensates, skin protectants, film formers, preservatives, and the like.

Examples of suitable pigments include, but are not limited to, iron oxides. Suitable builders for use in the compositions according to the invention include, but are not limited to, citrates, pyrophosphates, polyphosphates. Particularly useful electrolytes include but are not limited to inorganic chlorides and inorganic sulphates. These salts are preferably present 0%-10%, more preferably 0% - 5% and most preferably 0% - 3%, based on total weight of the compositions.

The compositions of the present invention may be directed applied to the skin or may be applied onto other delivery implements such as wipes, sponges, brushes, and the like. The compositions may be used in products designed to be left on the skin, wiped from the skin, or rinsed off of the skin. Further, the ringing gel compositions can be in the form of a gel, a bath, a wash, a mousse, a shampoo, a rinse, a lotion, a cream, a wipe, a brush, a sponge, or a spray.

The composition according to the invention may be prepared in accordance with the procedure described in PCT application no. PCT/EP00/05341, filed June 9, 2000 and Great Britain Patent Application No. 99133082, filed June 10, 1999, the disclosures of which are hereby incorporated by reference. Generally, the compositions of the present invention can be prepared by well-known mixing or blending procedures well known in the art, such as a mechanically stirred propeller, paddle and the like. The surfactant phase and the oil phase are preferably separately prepared with all of the components contained in the appropriate phase. The ringing gel composition is then formed normally by adding the oil phase to the surfactant phase with agitation and heat and the emulsion should be cooled down to form a gel.

Generally, the compositions according to the invention are prepared by preparing a premix of oil phase components, including for example, oil soluble benefit agents and oil soluble surfactants and/or emulsifiers. The ringing gel compositions according to the invention can contain water-insoluble and oil-insoluble benefit agents as well as water-soluble and oil-soluble benefit agents. Generally, if the benefit agent is a water-soluble component, it is present in the surfactant phase of the ringing gel composition. Likewise, if the benefit agent is oil-soluble, it is present in the oil phase of the ringing gel composition. Examples of water insoluble particulate solids suitable for incorporation into the ringing gel compositions of the invention include talc, clays, polymer beads, sawdust, and silica. seeds, ground nut shells, and dicalcium phosphate, pearlizers such as mica or glycerol or ethylene glycol mono-or di-stearate, glitter additives, and sunscreens such as titanium dioxide and zinc oxide. Such water insoluble ingredients may be dispersed in the hot emulsion of surfactant phase and oil phase prior to formation of the ringing gel. Porous particles (so called micro-sponges) containing absorbed active ingredients or gelatin or other microcapsules may be suspended in the oil phase. Other benefit agents which may be suspended include insect repellents and topical pharmaceutical preparations, e.g., preparations for the treatment of acne, fungicides for athlete's foot, ringworm, antiseptics, or antihistamines.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

### EXAMPLES

### A. Preparation of Ringing Gel Compositions

The formulations of Examples 1 - 6 were made according to the following procedure:
a premix of oil phase components, including salicylic acid and oil soluble surfactants or emulsifiers were added to a mixing vessel;
water was then charged to a separate main mixing vessel and the mixture was heated to 60°C;
any water soluble salts or materials such as humectants were added to water with mixing and aqueous surfactants were added to form the surfactant phase;
the temperature of the main vessel was maintained at 60°C;
the oil phase premix was then added to the main vessel containing the surfactant phase and dispersed with cooling to form a homogenous emulsion.
additional nonionic surfactant and/or water was added to the mixture at this stage if necessary to improve clarity of the product.

The mixture was then cooled to form a gel.

The registered trademarks noted below have the following significance:
EMPICOL® - CVN is a C₈ alkyl ether carboxylic acid available from Albright & Wilson.
EMPICOL® LB40 is a C₈-C₁₀ alkyl sulfate available from Albright & Wilson.
EMPICOL® 0251/70J is a C₁₂₋₁₄ alkyl 3 mole ethoxy sulfate available from Albright & Wilson.
EMPICOL CED 5 is C₁₂ alkyl 5 mole ethoxy carboxylate available from Albright & Wilson.
EMPICOL CED 5S is sodium laureth carboxylate available from Albright & Wilson.
EMPICOL 0758 is a C₁₀ alkyl sulfate available from Albright & Wilson.
EMPIGEN® BB is a C₁₂₋₁₄ alkyl betaine available from Albright & Wilson.
EMPIGEN® CDL is a coconut ampho acetate available from Albright & Wilson.
EMPIGEN CDL 30/J/35 is sodium C₁₂ amphoacetate available from Albright & Wilson.
EMPILAN® KB2 is a C₁₂₋₁₄ alcohol 2 mole ethoxylate available from Albright & Wilson.
EMPILAN® KB6 is a C₁₂₋₁₄ alcohol 6 mole ethoxylate available from Albright & Wilson.
EMPILAN KB12 is C₁₂₋₁₄ alcohol 12 mole ethoxylate available from Albright & Wilson.
GLUCAPON® 215CS is a C₈₋₁₀ alkyl polyglucoside D.P. 1.5 available from Cognis.
KATHON® CG is a biocide which is a mixture of methylchloroisothiazolinone and methylisothiazolinone available from Rohm & Haas.
CERAPHYL GA-D is maleated soybean oil available from International Specialty Products.
DC 556 SILICONE FLUID is phenyl trimethicone available from Dow Chemicals.
FLORASOLV S PEG 10 SUNFLOWER PEG-10 is sunflower tryglycerides available from Floratech International.
KRISTOL M14 is a light mineral oil having a visocisty of about 14 centistokes.
KRISTON M70 is a heavy mineral oil having a viscosity of about 20 centistokes.
ESTOL IPM 1512 is isopropyl myristate available from Uniqema.
MIGLYOL 812 S is capric/caprylic triglycerides available Condea Chemie.
ARLASOLV DMI is dimethyl isosorbide available from Uniqema.

### B. Test Method

The following test method was used in the Examples:

### 1. Salicylic Acid Test Protocol

A spectrofluorometer known as Skin Skan, manufactured by Instruments S.A., Inc., Jobin Yvon/Spex Division, was used to measure the amount of salicylic acid deposited on the skin of volunteers who had washed their forearms using the wash protocol outlined below. The Skin Skan focused a monochromatic beam of light onto the surface under investigation. The resulting fluorescence was measured. The change in fluorescence spectra at 306 nm and 421 nm was used to measure salicylic acid concentration on the forearms of volunteers.

### 2. Wash Protocol:

### a. HAND PRE-WASH:

- Use 100°F water: wash the inner (Volar) forearms on both hands of the panelists with *Neutrogena's Non-Drying Cleanser.* Rinse thoroughly and blot dry with paper towels.
- Go to Skin Skan Base reading. Take Baseline Readings on each site.
- Proceed to Hand Wash.

### b. HAND WASH:

- Mark off 2 x 3" area on each arm to localize the wash to the inner forearms. Mark also a circle 0.75" in diameter and cover it with a protective adhesive bandage. A representative example would be the use of Band-Aid Brand Waterblock Plus Adhesive Bandage..
- Be sure that the arm is no more than 1" from the counter top and at a distance of 6 inches from the spout of the faucet. Also be sure to hold arm directly below the faucet.
- Wet forearms for 5 seconds with 100°F water.
- Apply 0.5 cc of test sample to the marked area and rub into a lather for 10 seconds.
- Allow the lather to remain on the skin for additional 15 seconds.
- Rinse the test area for 15 seconds.
- Pat dry (without rubbing) using paper towels.
- Deposit Salicylic Acid standard solution (∼10 µl of 0.2%) in one of the circles, which was protected with the adhesive bandage (internal standard)
- Read fluorescence by Skin Skan of the tested & standard containing circles.

### EXAMPLE 1

The following formulas were made in accordance with the teachings of this invention:

| **Formulation (% by wt.)** | **A** | **B** |
|---|---|---|
| *Surfactant Phase* | | |
| EMPICOL 0251/70J | 10.29 | 0.0 |
| EMPIGEN CDL 30/J/35 | 0.0 | 9.51 |
| EMPIGEN BB | 2.94 | 7.28 |
| EMPICOL CED 5 S | 5.65 | 0.0 |
| EMPICOL 0758 | 0.0 | 1.94 |
| EMPILAN KB12 | 4.90 | 0.0 |
| EMPILAN KB6 | 0.0 | 0.0 |
| EMPILAN KB2 | 6.86 | 7.77 |
| Sodium Chloride | 1.96 | 0.0 |
| Glycerol | 1.96 | 0.0 |
| 47% NaOH | 0.20 | 0.0 |
| EDTA | 0.10 | 0.10 |
| Citric Acid | 0.20 | 0.19 |
| Sodium Benzoate | 0.29 | 0.29 |
| Water | 38.18 | 46.71 |

| *Oil Phase* | | |
|---|---|---|
| Salicylic Acid | 1.96 | 1.94 |
| KRISTOL M70 | 24.51 | 24.27 |
| Viscosity (cs) of Oil Phase | 70 | 70 |
| HLB of Oil Phase | 11 | 11 |
| *Salicylic Acid Deposition* | 2.5 ug/cm2 | 1.2 ug/cm2 |

As demonstrated by this example, by varying the amount and types of surfactant the amount of deposition can be significantly increased. When formulation A, containing less amphoteric surfactant, was compared to formulation B, the amount of deposition for formulation A was significantly increased.

### EXAMPLE 2

| **Formulation (% by wt.)** | **C** | **D** |
|---|---|---|
| *Surfactant Phase* | | |
| EMPICOL 0251/70J | 10.24 | 10.59 |
| EMPIGEN BB | 2.93 | 3.03 |
| EMPICOL CED 5 S | 5.62 | 5.81 |
| Sodium Chloride | 1.95 | 2.02 |
| Glycerol | 1.95 | 2.02 |
| 47% NaOH | 0.20 | 0.20 |
| EDTA | 0.10 | 0.10 |
| Citric Acid | 0.20 | 0.20 |
| Sodium Benzoate | 0.29 | 0.30 |
| EMPILAN KB2 | 4.88 | 5.05 |
| EMPILAN KB12 | 4.88 | 5.05 |
| Water | 47.73 | 44.64 |

| *Oil Phase* | | |
|---|---|---|
| Salicylic Acid | 1.95 | 2.02 |
| MIGLYOL 812S | 4.88 | 5.41 |
| KRISTOL M70 | 12.20 | 13.51 |
| Viscosity (cs) of Oil Phase | 55 | 70 |
| HLB of Oil Phase | 11 | 11 |
| *Salicylic Acid Deposition* | 1.6 ug/cm2 | 2.3 ug/cm2 |

As demonstrated above, a significant increase was observed in the salicylic acid deposition with formulation D having an extra 2% of Oil. While not wishing to be bound by theory, this increase in oil level is expected to increase the availability of the oil/salicylic acid mixture to the surface of the skin. It also could be due to changes in the viscosity of the oil phase and the surfactant packing phase, which would also increase the availability of the oil/salicylic acid mixture to the surface of the skin.

### EXAMPLE 3

| **Formulation (% by wt.)** | **E** | **F** |
|---|---|---|
| *Surfactant Phase* | | |
| EMPICOL 0251/70J | 10.24 | 8.88 |
| EMPIGEN BB | 2.93 | 2.54 |
| EMPICOL CED 5 S | 5.62 | 4.87 |
| Sodium Chloride | 1.95 | 1.69 |
| Glycerol | 1.95 | 1.69 |
| 47% NaOH | 0.20 | 0.17 |
| EDTA | 0.10 | 0.08 |
| Citric Acid | 0.20 | 0.17 |
| Sodium Benzoate | 0.29 | 0.25 |
| EMPILAN KB12 | 4.88 | 4.23 |
| EMPLIAN KBE2 | 4.88 | 4.23 |
| Water | 47.73 | 52.43 |

| *Oil Phase* | | |
|---|---|---|
| Salicylic Acid | 1.95 | 1.69 |
| CCT oil | 4.88 | 4.88 |
| HMO oil | 12.20 | 12.20 |
| Viscosity (cs) of Oil Phase | 55 | 55 |
| HLB of Oil Phase | 11 | 11 |
| *Salicylic Acid Deposition* | 1.9 ug/cm2 | 2.2 ug /cm2 |

As demonstrated above, a significant increase was observed in the salicylic acid deposition with the sample with the additional added water.

### EXAMPLE 4

| **Formulation (% by wt.)** | **G** | **H** |
|---|---|---|
| *Surfactant Phase* | | |
| EMPIGEN CDL 30/J/35 | 9.51 | 8.24 |
| EMPIGEN BB | 7.28 | 5.88 |
| EMPICOL 0758 | 1.94 | 1.96 |
| EDTA | 0.10 | 0.10 |
| Citric Acid | 0.19 | 0.20 |
| Sodium Benzoate | 0.29 | 0.29 |
| EMPILAN KB2 | 7.77 | 6.86 |
| EMPILAN KB6 | 0.00 | 4.90 |
| Water | 46.71 | 45.10 |

| *Oil Phase* | | |
|---|---|---|
| Salicylic Acid | 1.94 | 1.96 |
| CCT | 0.00 | 0.00 |
| HMO | 24.27 | 24.51 |
| Viscosity (cs) of Oil Phase | 70 | 70 |
| HLB of Oil Phase | 11 | 11 |
| *Salicylic Acid Deposition* | 0.6 | 1.4 ug/cm2 |

As demonstrated above, a significant increase was observed in the salicylic acid deposition with the sample with the additional Empilan KB6.

### EXAMPLE 5

| **% w/w** | **I** | **J** |
|---|---|---|
| *Oil Phase* | | |
| Salicylic Acid | 0.87 | 0.85 |
| Kristol M14 | | 8.48 |
| Kristol M70 | 13.06 | |
| Ceraphyl GA-D | 4.35 | |
| DC 556 Silicone Fluid | | 8.48 |
| Viscosity (cs) of Oil Phase | 60 | 18 |
| HLB of Oil Phase | 10 | 8 |

| *Surfactant Phase* | | |
|---|---|---|
| EMPICOL 0251/70J | 15.67 | 15.27 |
| EMPIGEN BB | 8.71 | 8.48 |
| EMPILAN KB 2 | 7.56 | 8.30 |
| EMPILAN KB 12 | 3.92 | 2.97 |
| GLUCAPON CS 215 UP | 6.97 | 6.78 |
| Sodium Benzoate | 0.26 | 0.25 |
| Water | 38.63 | 59.86 |
| *Saticytic Acid Deposition* | 0.8 ug/cm2 | 0.8 ug/cm2 |

As demonstrated above, there were no dramatic differences in salicylic acid deposition using the different oil phases. This example shows that there is flexibility and choice in the oil phase, and still retain some level of deposition.

### Example 6

| **% w/w** | **K** | **L** | **M** | **N** |
|---|---|---|---|---|
| *Oil Phase* | | | | |
| Salicylic Acid | 2.0 | 2 | 2 | 2 |
| Kristol M14 | 20 | | 10.0 | |
| Kristol M70 | | | | 18 |
| Isopropyl Myristate | | 20 | | |
| FLORASOLVS PEG-10 SUNFLOWER | 2.0 | 2 | 2 | 2 |
| Myglyol 812S Capric/Caprylic Triglycerides | | | 10.0 | |
| Viscosity (cs) of Oil Phase | 14 | 10 | 22 | 70 |
| HLB of Oil Phase | 11 | 11 | 8 | 11 |

| *Surfactant Phase* | | | | |
|---|---|---|---|---|
| EMPICOL 0251/70J | 17.2 | 17.2 | 15.7 | 14.3 |
| EMPIGEN BB | 10.0 | 10 | 10.0 | 10 |
| EMPILAN KB 2 | 3.0 | | 3.5 | 5 |
| EMPILAN KB 12 | | | | 5 |
| EMPICOL CED 5 FL | 5.0 | 6 | 6.0 | 6 |
| EMPILAN KB 6 | 3.0 | 5 | 3.5 | |
| Glycerol | 1.0 | 1 | 1 | 1 |
| Sodium Hydroxide | 1.2 | 1.2 | 1.2 | 1.2 |
| Sodium Benzoate | 0.3 | 0.3 | 0.3 | 0.3 |
| Water | 32.1 | 33.3 | 31.6 | 32.0 |
| Sodium Chloride | 3.0 | 2 | 3 | 3 |
| Citric Acid | 0.2 | 0.2 | 0.2 | 0.2 |
| *Salicylic Acid Deposition* | 0.9 ug/cm2 | 1.6 ug/cm2 | 2.4 ug/cm2 | 2.3 ug/cm2 |

These examples show that there is a significant effect of the amount of salicylic acid deposition, depending upon the nature of the oil phase in the product. Comparing formulations K and L show that moving to a higher viscosity oil phase increases the amount of salicylic acid deposition. This is probably due to the greater resistance to reemulsification of the oil phase/ salicylic acid mixture, which would enhance deposition on the surface. A comparison of formulations M and N, demonstrate that the polarity of the oil phase affects deposition, with the less polar oil phase enhancing additional deposition of the mixture to the skin.

## Claims

1. A topically-applicable ringing gel composition comprising (a) a surfactant phase; (b) an oil phase; and (c) a benefit agent for depositing said benefit agent on a keratinous surface.

2. The composition of claim 1, wherein the surfactant phase comprises at least one amphoteric surfactant, at least one nonionic surfactant and at least one anionic surfactant.

3. The composition of claim 2, wherein:
(a) the amphoteric surfactant is selected from alkyl amphocarboxylates, alkyl betaines, amidoalkyl betaines, amidoalkyl sultaines, alkyl amphophosphates, alkyl phosphobetaines, alkyl pyrophosphobetaines, alkyl sulfobetaines, carboxyalkyl alkyl polyamines, and mixtures thereof;
(b) the nonionic surfactant is selected from alcohol ethoxylates, alkyl phenol ethoxylates, fatty acid ethoxylates, fatty acid monoalkylolamide ethoxylates, fatty alcohol propoxylates, fatty amine alkoxylates, fatty acid glyceryl ester ethoxylates, and mixtures thereof;
(c) the anionic surfactant is selected from alkyl sulfates; alkyl ether sulfates; alkyl monoglyceryl ether sulfates; alkyl monoglyceride sulfates; alkyl monoglyceride sulfonates; alkyl sulfonates; alkylaryl sulfonates; alkyl sulfosuccinates; alkyl ether sulfosuccinates; alkyl sulfosuccinamates; alkyl amidosulfosuccinates; alkyl carboxylates; alkyl ether carboxylates; alkyl amidoethercarboxylates; alkyl succinates; fatty acyl sarcosinates; fatty acyl amino acids; fatty acyl taurates; fatty alkyl sulfoacetates; alkyl phosphates; alkyl isethionates, and mixtures thereof.

4. The composition of any one of claims 1 to 3, wherein the oil phase has an HLB raging from 3 to 18, preferably from 8 to 11.

5. The composition of any one of claims 1 to 4, wherein the oil phase is selected from the group consisting of mineral oil, silicone oil, perfluorocarbons, alkyl esters and mixtures thereof.

6. The composition of any one of claims 1 to 5, wherein the oil phase has a viscosity ranging from 1 to 500 centistokes, preferably from 10 to 100 centistokes.

7. The composition of any one of claims 1 to 6, which comprises (a) from 60 to 95% by wt. of the surfactant phase, based on the total composition; and (b) from 5 to 40% by wt. of oil the phase, based on the total composition.

8. The composition of any one of claims 1 to 7, which comprises from 20 to 70 wt. % water, based on the total composition.

9. The composition of any one of claims 1 to 7, wherein the benefit agent is selected from the group consisting of selected from the group consisting of vasoconstrictors, collagen enhancers, anti-edema agents, depigmentation agents; reflectants; detangling/wet combing agents; film forming polymers; humectants; amino acid agents; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents; antihistamines; antiinfectives; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; skin firming agents, hair conditioners; hair softeners; hair moisturizers; vitamins; tanning agents; skin lightening agents; antifungals; depilating agents; shaving preparations; external analgesics; perfumes; fragrances counterirritants; hemorrhoidals; insecticides; poison ivy products; poison oak products; burn products; anti- diaper rash agents; prickly heat agents; make-up preparations; vitamins; amino acids and their derivatives; herbal extracts; retinoids; flavenoids; sensates; anti-oxidants; skin conditioners; hair lighteners; chelating agents; cell turnover enhancers; coloring agents; pigments; sunscreens and mixtures thereof.

10. The composition of claim 9 wherein the benefit agent is an anti-acne agent, such as benzoyl peroxide, retinol, elubiol, an antibiotic, salicylic acid or a mixture thereof.
